# EUROPEAN PATENT APPLICATION

(11) **EP 4 361 941 A1**
(43) Date of publication of application: **01.05.2024**
(21) Application number: 22204043.8
(22) Date of filing: 27.10.2022
(51) Int. Cl.: G06T 7/00, G06T 7/11

(54) **METHOD, PROCESSOR UNIT AND SYSTEM FOR PROCESSING OF IMAGES**

(71) Applicant: Carl Zeiss Meditec AG, 07745 Jena (DE)
(72) Inventor: Saita, Jayesh, 411040 Pune (IN); Panitz, Gerald, 73441 Bopfingen (DE); Chakroborty, Sandipan, deceased (IN)
(74) Representative: Gulde & Partner

(57) **Abstract**

The present invention relates to a method for processing of images (100), wherein the method comprises the steps of inputting the plurality of images (100) into a trained first neural network (201) and receiving, as an output of the first neural network (201) and for each of the plurality of images (100), at least one first classifier (201i) indicating a set image distortion type of the image, identifying (S200) a first subset (101) of the plurality of images (100) based on the first classifiers (201i), inputting the first subset of images (101) into a trained second neural network (202) different from the first neural network (201) and receiving, as an output of the second neural network (202) and for each of the first subset of images (101), at least one second classifier (202i) indicating a presence of a set tissue type in the image (101i), and identifying (S400) a second subset (102) of the first subset of images (101) based on the second classifiers (202i). The invention further relates to a processor unit and to a system for performing such method.

## Description

### TECHNICAL FIELD

The present invention relates to a method for processing images, particularly a method for processing medical images and, preferably, for filtering particularly relevant medical images and presenting those particularly relevant images to a practitioner in an advantageous manner. The present invention further relates to a processor unit configured to carry out such method as well as to a system configured for carrying out such a method and comprising such a processor unit.

### BACKGROUND OF THE INVENTION

The use of modern technology has become integral part of modern medicine. Various imaging techniques have been developed and are regularly used in diagnostics as well as in surgery. Therein, the use of these imaging techniques enables a better discrimination of various structures and tissues in the patient which can aid the diagnostic as well as a practitioner, or surgent, during a surgical procedure. Therein, a practitioner can not only better plan a surgical procedure based on obtained high-quality image data of a patient, but the image data can also support the practitioner during the actual performance of the procedure. Exemplarily, image information acquired during surgery can be overlaid with previously acquired diagnostic image data to indicate poorly visible tissue boundaries to the practitioner. Likewise, the image data can be used to control robotic surgical instruments or to perform certain operations in part or even fully.

During a surgical procedure, multiple practitioners might cooperate in order to perform the surgery, mostly the surgeon and a pathologist, wherein the latter often has to make histological decisions "on the spot" in order to guide the surgeon. In the past or conventionally, physical samples taken from a patient had to be sent from the operation room to the lab, where they had to be cut and stained, and then put under a microscope for review by the pathologist. However, with digital imaging techniques, image data can be obtained by the surgeon and can be send digitally and in real time to the pathologist for making a histological assessment. Therein, an image stream might be provided to the pathologist with a speed of image generation such that the pathologist receives and has to review a large amount of image data during a short time.

As the pathologist has to review a large amount of image data, there is only a limited amount of time for him/her to analyse the respective image, which involves the risk that the evaluation is incorrect. At the same time some of the obtained images may be defective as they have been obtained with imaging errors or might exhibit structures that are not helpful for the practitioner to make the relevant histological assessment. Nevertheless, browsing through the multitude of images takes a lot of time for the pathologist, while time might be a critical factor in the surgery. Exemplarily, the surgery can be a brain surgery and the images can be obtained with a confocal laser endomicroscopy, CLE.

While prior art solutions exist to help practitioners with reviewing image date, the known solutions disadvantageously can only inaccurately determine whether an image or image region is relevant for the pathologist and - even if such a determination is made - can often not provide the practitioner with an understanding of why the determination was made in that way. Due to such lack of understandable decision-making and a lack of interaction possibilities, the known solutions are often met with a lack of trust by experienced practitioners, thus leading to a lack of acceptance of these solutions and to the problem that practitioners ultimately perform the review themselves.

It is therefore an object of the present invention to obviate or reduce at least some of the drawbacks of the prior art and to provide an improved method for image processing that shall be usable to provide an improved assistance to medical practitioners, e.g., in time-critical situations.

### DESCRIPTION OF THE INVENTION

According to the present disclosure, a method for processing of images, a processor unit for processing images as well as a system comprising such processor unit are provided, that each overcomes or reduces at least some of the disadvantages of the prior art. Further preferred embodiments are subject-matter of the respectively depending claims.

A first aspect of the present disclosure relates to a method for processing of images. The method comprises the step of inputting a plurality of images captured using at least one optical device into a trained first neural network. In other words, the first trained neural network receives each of the plurality of images as an input, preferably each image as an individual input. The first trained neural network is preferably trained for automatic image recognition as described in more detail below and assigns, to each image that is input into the first trained neural network, a first classifier that is associated with a set image distortion type (e.g., one of a plurality of image distortion types) of that respective image and preferably provides a numerical value associated with a probability of the set image distortion type in that image. Hence, this step of the method further comprises receiving, as an output of the first neural network and for each of the plurality of images, at least one such first classifier indicating a set image distortion type of the respective image (i.e., a presence of a set image distortion in the respective image). Also, a plurality of first classifiers, each associated with a set image distortion type, can be received for a single image.

The method preferably comprises the step of obtaining the plurality of images that were captured using at least one optical device. The images are preferably obtained from the optical device directly or, indirectly, via at least one memory, via at least one processor unit for pre-processing and/or via at least one GPU or the like. The images are preferably stored as digital image files in a common image format and further preferably comprise pixel-wise information on a grey scale or a brightness value in at least one channel, further preferred in multiple channels, e.g., coding different colors. In other words, each of the images is preferably present as a bitmap connecting pixel coordinates with at least one greyscale value. Further preferred, each of the plurality of images is present in a same format. The at least one optical device preferably is a confocal laser endomicroscopy, CLE, unit configured to obtain images via confocal laser endomicroscopy. However other optical devices, such as a confocal microscope or a conventional surgical microscope or endoscope with a high-resolution camera can also be used to obtain the plurality of images.

In a next step of the method, a first subset of the plurality of images is identified based on the first classifiers. As set forth above, each of the first classifiers indicates a set image distortion type for the respective image and preferably indicates a probability of that set image distortion type being present in the respective image. Hence, based on the at least one first classifier output by the first trained neural network for each image input into the first trained neural network an assessment can be performed whether such an image distortion type is likely present in the respective image or not. Based on such an assessment, in this step of the method the images of the plurality of images are sorted into different subsets based on a probability that a set image distortion type is present, such that, for each subset, the probability of a set image distortion type fulfils a certain condition, e.g., that the probability of a set image distortion type is less than a given threshold. Preferably, the first subset of images are images without distortions.

A next step of the method comprises inputting the first subset of images into a trained second neural network that is different from the first neural network. The second trained neural network is preferably trained for automatic image recognition as described in more detail below and assigns, to each image that is input into the second trained neural network, a second classifier that is associated with a set image tissue type (e.g., one of a plurality of tissue types) of that respective image and preferably provides a numerical value associated with a probability of the set tissue type in that image. Hence, this step of the method further comprises receiving, as an output of the second neural network and for each of the first subset of images, at least one such second classifier that is indicating a (presence of a) set tissue type in the respective image. Also, a plurality of second classifiers, each for a set tissue type, can be received for a single image.

In a next step of the method, a second subset of the plurality of images is identified based on the second classifiers. As set forth above, each of the second classifiers indicates a set tissue type (being present) in the respective image and preferably indicates a probability of that set tissue type being present in the respective image. Hence, based on the at least one second classifier output by the second trained neural network for each image input into the second trained neural network an assessment can be performed whether such a tissue type is likely present in the respective image or not. Based on such an assessment, in this step of the method the images of the first subset of images are further sorted into different subsets based on a probability that a set tissue type is present, such that, for each subset, the probability of a set tissue type fulfils a certain condition, e.g., that the probability of a set tissue type exceeds a given threshold. Particularly, the second subset of images is a subset of images showing at least one tissue type.

The method of the present disclosure thus advantageously provides a two-step filtering for images obtained by an optical device, wherein a first filtering step pertains to filtering images based on image distortions and wherein a second filtering step pertains to filtering the pre-filtered images based on tissue types. It has been found that providing such two-step filtering process advantageously increases the filtering efficiency, as e.g., optically distorted pictures could not be misinterpreted as showing a different tissue type, and as a user, e.g., a pathologist or other practitioner, has more interaction possibilities with the filtering process, e.g., by adjusting a filtering intensity (efficiency) individually for each of the two filtering stages.

In the method of the present disclosure, preferably each image of the first subset of images is associated with a respective first classifier indicating one of the at least one set image distortion type (being present) with a probability below a set first threshold. As set forth above, each first classifier of the at least one first classifier is associated with a set image distortion type of at least one image distortion type. Preferably, each first classifier of a plurality of first classifiers indicates, for a given image, the presence of a specific image distortion type of a plurality image distortion types in the given image. As each image of the first subset of images is associated with a first classifier being below a set first threshold, each image of the first subset of images has a set image distortion type being present with a likelihood that is less than a set likelihood. Preferably, each image of the first subset of images is associated with a plurality of first classifiers and each of these first classifiers has a value below an associated first threshold, i.e., none of a plurality of image distortion types is present in the first subset of images. Advantageously, the first set of images can thus be controlled to not comprise any images that are not usable for further image recognition due to at least one present image distortion type.

Further preferred, in the method of the present disclosure, each image of the second subset of images is associated with a respective second classifier indicating a presence of one of the at least one set tissue type with a probability above a set second threshold. As set forth above, each second classifier of the at least one second classifier is associated with a set tissue type of at least one tissue type. Preferably, each second classifier of a plurality of second classifiers indicates, for a given image, the presence of a specific tissue type of a plurality of tissue types in the given image. As each image of the second subset of images is associated with a second classifier being above a set second threshold, each image of the second subset of images has a set tissue type being present with a likelihood that is above a set likelihood. Preferably, each image of the second subset of images is associated with a plurality of second classifiers and each of these second classifiers has a value above an associated second threshold, i.e., each of a plurality of tissue types is present in each image of the second subset of images. Alternatively, each image of the second subset of images is associated with at least one of a plurality of second classifiers and each of these second classifiers has a value above an associated second threshold, i.e., at least one of a plurality of tissue types is present in each of the second subset of images. Further preferred, pictures in the second subset of images comprise certain combinations of tissue types or exclude certain combinations of tissue types. Also preferred, each image of the second subset of images shows only one of a plurality of tissue types. Such filter characteristics can be advantageously set by setting the second classifiers and thresholds. Thus, the second filter stage advantageously filters the images for medical relevance.

In a particularly preferred embodiment, each first classifier indicates an image distortion type to be one of an optical distortion (such as motion artefacts, perturbations, or the like), a diminished signal-to-noise-ratio (dSNR), and an irregular brightness or contrast. Further preferred, a first classifier may also indicate that an image distortion type is a non-distortion, i.e., that an image without any distortions was input into the first trained neural network. Such a first classifier might be determined by the first trained neural network directly or as a function of the determined first classifiers related to the aforementioned image distortion types. In case of a first classifier indicating a non-distortion, the first subset of images preferably comprises images with such first classifiers having values above a predefined and corresponding first threshold. Hence, the first trained neural network advantageously filters out technically bad quality images and the first subset of images advantageously comprises only images in which none of the above artefacts are present but which are technically good and hence usable for a (medical) practitioner.

In a further preferred embodiment, each second classifier indicates a present tissue type being one of a hypercellular region, a necrotic region, a fibroid region, an adenoid region, and a giant cell region. Therein, a hypercellular region preferably is characterized by an increased number of cells compared to a normal cell number for a certain area of the body. A necrotic region preferably is characterized by localized area of dead tissue where normally is living tissue. A fibroid region is preferably characterized by non-cancerous growths structures that are made up of muscle and/or fibrous tissue. An adenoid region is preferably characterized by a growth of gland-like structures (a gland-like growth of structures), preferably of lymphoepithelial tissue. A giant cell region is preferably characterized by cells with an arc of nuclei toward an outer membrane, which are often formed by fusion of epithelioid cells, derived from macrophages. The second trained neural network advantageously filters out medically (ir-)relevant images and the second subset of images advantageously comprises only images in which at least one, exactly one, or a combination of the above tissue types is present. The output of the second trained neural network are images which are technically good and contain clinically relevant regions.

In a particularly preferred embodiment, at least one first classifier with the corresponding at least one first threshold is set based on a user input. By setting the first classifiers that are to be determined by the first trained neural network, a user can set whether the plurality of images should be filtered for image distortion and for which type(s) of image distortion they should be filtered. By setting the associated first thresholds, a user can further set the strength of image distortions that should be filtered. Exemplarily, when inputting an image with a weak image distortion to the first trained neural network, the network will regularly output a first classifier with a rather low value, hence by setting a rather high associated first threshold, such an image would not be filtered out, i.e., would still form part of the first subset of images.

Additionally or alternatively, at least one second classifier with the corresponding at least one second threshold are preferably set based on a user input. By setting the second classifiers that are to be determined by the second trained neural network, a user can set whether the plurality of images should be filtered for tissue types and for which tissue type(s) they should be filtered. By setting the associated second thresholds, a user can further set the strength of filtering. Exemplarily, by setting a rather low threshold, an image having a set tissue type only in a very small region or obscured by other tissue, for which the second trained neural network would determine a rather low value of the associated second classifier, would still be identified as a part of the second subset of images. On the contrary, by setting a rather high threshold, such an image would be filtered out and would not be part of the second subset of images.

In a particularly preferred embodiment, at least one of the first neural network and the second neural network is a convolutional neural network, CNN, using depthwise separable convolution, linear bottleneck layers and/or inverted residual layers. In other words, despite being distinct neural networks that are distinctively trained, that receive distinct inputs (plurality of images for the first trained neural network and first subset of images for the second trained neural network) and that provide distinct outputs (first subset of images for the first trained neural network and second subset of images for the second trained neural network), the first neural network and the second neural network preferably have a same or at least similar structure. Preferably, the first and/or second neural network uses a Mobilenetv2 structure, but other structures are possible.

Particularly preferred, the first and/or second neural network use depthwise separable convolution. Therein, at least one convolution operator in the neural network is replaced by the product of two more simple operators. Advantageously, if a two-dimensional convolution operation is separated into two one-dimensional filters, the computational costs on an *N × M* image with a *m x n* filter reduces from a magnitude of *(M*N*m*n)* to a magnitude of *(M*N*(m+n)).* Particularly, in the first and/or second neural network a standard convolution operator is separated into a so-called depthwise convolution and a so-called pointwise 1 × 1 convolution. Therein, for an image with *M* channels (depth) and a spatial extension of *X* and *Y*, the depthwise convolution preferably applies *M* single filters to each input channel, i.e., producing *M* outputs. The pointwise convolution then applies a 1×1 convolution (with *X→1, Y→1, and M→M)* to combine the outputs of the depthwise convolution. In contrast to a standard convolution that filters and combines inputs into a new set of outputs in one step, such depthwise separable convolution splits it into two layers, one for filtering and one for combining, wherein this factorization again has the effect of reducing computation costs and the model size.

Further preferred, the first and/or second neural network use linear bottleneck layers. Therein, a linear bottleneck layer is a hidden layer of the neural network that has a dimensionality which is smaller, preferably much smaller, than a dimensionality of surrounding layers. Such a linear bottleneck layer advantageously filters non-linearities in the inputs and/or outputs. Particularly preferred, a linear bottleneck layer is positioned between a first plurality of layers for reducing dimensions of an input and a second plurality of layer for increasing dimensions of an input.

Further preferred, the first and/or second neural network use inverted residual layers. Therein, the fundamental idea of a residual is to provide the output of a layer of the neural network directly to a subsequent but not adjacent layer, i.e., providing to that subsequent layer an input coming from the precedent and adjacent layer as well as an output of said upstream layer. Such skip connections advantageously improve gradient propagation across multiple layers and the performance in the training of the neural network. In the inverted residual layer, the skip connection is not applied between full-dimensional layers surrounding a bottleneck layer but the skip connection is applied between bottleneck layers surrounding higher-dimensional layers.

In a particularly preferred embodiment, the method of the present disclosure further comprises the step of inputting the second subset of images into a trained third neural network different from the first neural network and the second neural network. Therein, the third neural network is preferably not only trained and applied differently than the first and second neural network, but the third neural network preferably has a different structure than the first and second neural network. Further, in this embodiment, the method comprises the step of receiving, as an output of the third neural network and for each of the second subset of images, a segmentation map indicating at least one image segment comprising one of the at least one set tissue type in the respective image. In other words, the third neural network does not filter images according to whether or not they comprise certain tissue types, but it rather provides information on the locations of and boundaries between certain tissue types in a given image input therein.

Particularly preferred, the segmentation map determined per image by the third neural network comprises, for each pixel or group of pixels of the respective image, at least one third classifier related to a presence of one of the at least one set tissue type in the respective pixel or group of pixels. Therein the at least one set tissue type preferably is one of the at least one tissue types filtered by the at least one second classifiers. In other words, the third neural network is preferably configured to determine, for each pixel or plurality of pixels, the presence of a given tissue type of the tissue types filtered by the second trained neural network. This correspondence between the at least one second and the at least one third classifier and by inputting the second subset of images in the third trained neural network, segmentation maps are only provided for images that are already filtered to comprise the specific tissue type that shall be located in the segmentation map. This advantageously reduces the processing time. Further advantageously, compared to back propagation in the second trained neural network, using the third trained neural network with the different structure, segmentation maps can be provided for the images of the second subset of images with a better quality as any comparable map (e.g., a heat map) that could be provided by the second trained neural network itself.

The method of this embodiment preferably further comprises the step of displaying an image of the second subset of images with at least one visually highlighted image segment comprising one of the at least one set tissue type based on the segmentation map. In other words, the visually highlighted image segment is determined based on the segmentation map and preferably by visually highlighting pixels or group of pixels that are associated with a third classifier having a value above a set third threshold. Therein, a value of the third classifier above the third threshold indicates that a set tissue type is present in a pixel or group of pixels having such value of an associated third classifier. Further, a segmentation map may comprise, per pixel or group of pixels, a plurality of third classifiers and hence multiple visually highlighted image segments may correspond to multiple third classifiers, i.e., different tissue types, being present in the segments. According to this embodiment, only the relevant images previously filtered from the second trained neural network are displayed and the reason for the assessed relevance of these images is indicated by the visually highlighted segments. Hence, the relevance of these images can be justified to and evaluated by the practitioner.

Particularly preferred, the third neural network is a fully convolutional neural network, FCN, that comprises a contracting subnetwork followed by an expansive subnetwork. Therein, the contracting network comprises a plurality of pooling operators and a contracting path of an image in the contracting subnetwork consists of the repeated application of convolution operations ("down-convolution") and pooling operations. Further, the expansive subnetwork comprises a plurality of upsampling operators and an extensive path of an image in the expansive subnetwork consists of the repeated application of an upsampling operation and convolution operations ("up-convolution"). Therein, at each pooling step the number of feature channels of an image in the contracting subnetwork is increased, while at each convolution operation in the extensive path of an image in the expansive subnetwork the number of feature channels is decreased. The third neural network further comprises a plurality of concatenations that connect each layer of the contracting subnetwork with a corresponding layer of the expansive subnetwork and provide additional input for the upsampling (as a skip connection). Preferably, the third neural network has a U-Net structure, but other structures are possible.

The method of the present disclosure provides an improved processing, particularly filtering, of images, preferably medical images, for finding relevant images. Therein, the method of the present disclosure does not only filter unusable images, but also filters usable but irrelevant images, thus only leaving usable and relevant images. By providing such filtering of images, a practitioner is confronted with a significantly reduced number of images and can thus spend more time on analysing individual images. Further, in the preferred embodiment using a third trained neural network, a justification for the relevance of the filtered images is provided by graphically highlighting the relevant regions found in the image, thus providing an explicit indication of the relevance of these images. Further, the system does not only elaborate its decision-making to classify an image as relevant by graphically highlighting the clinically relevant regions found in the image, but it can also classify the highlighted regions into different clinically relevant categories thus allowing the practitioner to quickly assess the sizes of different highlighted and classified regions as well as the distributions of regions present in images. For example, necrotic regions are present in fast growing tumors such as glioblastoma multiforme (GBM), medulloblastoma, and secondary tumors of the central nervous system (CNS), while adenoid regions are typically present in metastasized lung cancers or other adenoid cancers. By visually highlighting and classifying these tissue types in the images of the second subset of images, an analysis of the practitioner is supported.

In the method of the present disclosure, the first neural network preferably comprises a plurality of first neural subnetworks, wherein each of these first neural subnetworks receives the plurality of images and outputs, for each of the plurality of images, one of a plurality of first classifiers with a respective probability. Therein, the plurality of first classifiers relates to a plurality of image distortion types and a probability refers to a value assigned by the respective neural network to indicate a certainty of the network that the respective distortion type was detected in an image. In other words, each of the first neural subnetworks is a neural network trained to identify one of a plurality of image distortion types in an input image and to output a corresponding classifier.

Further preferred, in the method of the present disclosure, the second neural network comprises a plurality of second neural subnetworks, wherein each of these second neural subnetworks receives the first subset of images and outputs, for each of the first subset of images, one of a plurality of second classifiers with a respective probability. Therein, the plurality of second classifiers relates to a plurality of tissue types and a probability refers to a value assigned by the respective neural network to indicate a certainty of the network that the respective tissue type was detected in an image. In other words, each of the second neural subnetworks is a neural network trained to identify one of a plurality of tissue types in an input image and to output a corresponding classifier.

Further preferred, the third neural network comprises a plurality of third neural subnetworks, wherein each of these third neural subnetworks receives the second subset of images and outputs, for each of the subset of images, a segmentation map indicating at least one image segment comprising one of a plurality of set tissue types (one of the at least one set tissue type). Therein, the segmentation map comprises, for each pixel or group of pixels of the respective image, one of a plurality of third classifiers related to a presence of the one of the plurality of set tissue types in the respective pixel or group of pixels. In other words, each of the third neural subnetworks is a neural network trained to locate one of a plurality of tissue types in an input image and to output a corresponding segmentation map.

By applying multiple first, second, and/or third neural subnetworks, the filtering efficiency is advantageously further improved as individually and more specifically trained neural networks usually require less layers to provide a reliable classification. Hence, in this embodiment, "boosting" by applying three distinct trained neural networks is combined with "bagging" by using multiple parallel subnetworks in each stage for advantageously providing highest performance. Further, using multiple first, second, and/or third neural subnetworks also increases the interactivity of the method of the disclosure, allowing a user to individually select certain first, second, and/or third classifiers (i.e., to (de-)activate certain first, second, and/or third neural subnetworks) and/or to individually adjust certain first, second, and/or third thresholds (i.e., to set a filtering strength in the first and/or second network or to set a segmentation strength in the third network). Hence, compared to solutions with one fixed (probability) threshold for a whole process, where a user has no control over the output of the individual neural networks and subnetworks, in the method of the present disclosure the user is advantageously given the full control of the multistage filtration process and of the subsequent visualisation.

Further preferred, in the method of the present disclosure, the first classifier(s), the second classifier(s) and/or the third classifier(s) are set based on a user input. Further preferred, the first threshold(s), the second threshold(s) and/or the third threshold (s) are set based on a user input.

Hence, in this preferred embodiment all stages (first, second, third) are optional in nature and the user advantageously has the control on enabling or disabling any of these stages. Particularly advantageous by providing first/second probability thresholds for the first/second neural network, a user can control the output of these neural networks, which gives the user the ability to increase or decrease the strength of the filtration process in the first/second network. Further advantageous, the user can freely decide on the visual highlighting he is interested in by setting the third classifiers accordingly and can also adjust the visualisation strength (or certainty that highlighted structures are indeed the classified structures) by setting the third thresholds.

Another aspect of the present disclosure relates to a processor unit that is configured for performing the method according to the present disclosure. A processor unit according to the present disclosure comprises one or a plurality of processors. The one or plurality of processors may include at least one of a central processing unit (CPU), a graphic processing unit (GPU), and a neural processing unit (NPU). Hardware may include one or more of an accelerator or a machine learning accelerator. A processor unit according to the present disclosure may refer to a single-core processor, a multi-core processor, to at least one core of a multi-core processor or to cores of different single- or multi-core processors but is not limited to any of the above.

Further, any electronic, electric devices and/or any other relevant devices or components according to embodiments of the present disclosure, except those described explicitly as hardware, may be implemented utilizing any suitable hardware, firmware (e.g. an application-specific integrated circuit), software, or a combination of software, firmware, and hardware. For example, the various components of these devices may be formed on one integrated circuit (IC) chip or on separate IC chips. Further, the various components of these devices may be a process or thread, running on one or more processors, in one or more computing devices, executing computer program instructions and interacting with other system components for performing the various functionalities described herein. The computer program instructions may be stored in a memory and may be implemented in a computing device using a standard memory device, such as, for example, a random-access memory (RAM). The computer program instructions may also be stored in other non-transitory computer readable media.

A person of skill in the art should recognize that the functionality of various computing devices may be combined or integrated into a single computing device, or the functionality of a particular computing device may be distributed across one or more other computing devices without departing from the scope of the exemplary embodiments of the present invention. Therein, the multiple computing devices may be co-located or may be located in different locations and in a distributed manner. In case of distributed computing devices, suitable communication means are established between the computing devices for transmitting between these computing devices data required for carrying out the respective steps or for providing the respective functions of the individual computing devices. Many possibilities exist for distributing the steps or functions of the present disclosure onto multiple computing devices without departing from the scope of invention.

Particularly, the processor unit according to the invention may comprise a first processor unit that is configured for performing the operations related to the first neural network, particularly for the inputting of the images into the trained first neural network and for receiving, as outputs, the first classifiers. Further, the processor unit according to the invention may comprise a second processor unit that is configured for performing the operations related to the second neural network, particularly for the inputting of the first subset of images in to the trained second neural network and for receiving, as outputs, the second classifiers. Further, the processor unit according to the invention may comprise a third processor unit that is configured for performing the operations related to the third neural network, particularly for the inputting of the second subset of images in to the trained third neural network and for receiving, as outputs, the classification maps. The first, second and third processor units may be located at the same or at different locations. If the first, second and third processor units are distributed over different locations suitable communication means are established between the first, second and third processor units, for example a local bus system, a local network and/or or a long-distance communication network. Further preferred, the first, second and third processor units are also configured for performing the training of the first, second, and third neural networks, respectively. However, the training of the first, second, and third neural networks may also be performed by different processor units.

The method of the present disclosure may be performed in a localized or a distributed manner and another aspect of the present disclosure relates to a method for processing of images, wherein the method comprises a first step of identifying a first subset of a plurality of images, wherein the first subset of images is based or is identified based on first classifiers that were output by a trained first neural network, wherein a first classifier was output by the trained first neural network for each of the plurality of images and wherein the first classifiers are related to image distortion types of these images. The first step of this method may thus be carried out by a processor unit that is different from a processor unit operating the trained first neural network and that receives the first classifiers or the first subset of images from the processor unit operating the trained first neural network. Said method further comprises another step of inputting the first subset of images into a trained second neural network that is different from the trained first neural network and the step of receiving, as an output of the second neural network and for each of the first subset of images, at least one second classifier indicating a presence of a set tissue type in the image. The method comprises another step of identifying a second subset of the first subset of images based on the second classifiers. The method of the present disclosure may be performed in a localized or a distributed manner and another aspect of the present disclosure relates to a method for processing of images, wherein the method comprises a first step of inputting a first subset of a plurality of images into a trained second neural network that is different from a trained first neural network, wherein the first subset of images is identified based on first classifiers that are (have been) output by the trained first neural network with respect to each of the plurality of images. The first step of this method may thus also be carried out by a processor unit that is different from a processor unit operating the trained first neural network and that receives the first classifiers or the first subset of images from the processor unit operating the trained first neural network. The method further comprises the step of receiving, as an output of the second neural network and for each image of the first subset of images, at least one second classifier. The method further comprises the step of identifying a second subset of the first subset of images based on the second classifiers. Therein, the first classifiers are related to image distortion types of the plurality of images and the second classifiers are related to a presence of a set tissue type in the first subset of images. Preferred embodiments of the methods performed in a distributed manner according to these aspects of the present disclosure correspond to the preferred embodiments of the method as described above.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly defined so.

Another aspect of the present disclosure relates to a system for processing images, particularly medical images, the system comprising a confocal laser endomicroscopy, CLE, unit that is configured for capturing a plurality of CLE images. Confocal laser endomicroscopy, CLE, is an endoscopic modality developed to obtain very high magnification and resolution images and is based on tissue illumination with a low-power laser and a subsequent detection of the fluorescence of light reflected from the tissue through a pinhole. The CLE unit may be configured as a probe-based CLE, pCLE, unit comprising a fiberoptic bundle with an integrated distal lens that is connected to a laser scanning unit. The CLE unit may also be configured as endoscope-based CLE, eCLE, unit that uses a confocal microscope that is integrated into the distal tip of a conventional endoscope, wherein the tip may comprise the (MEMS) scanner and the optics, and wherein a laser might be included in a base unit connected to the endoscope unit..

The system further comprises a memory that is configured for storing at least one trained neural network. Preferably, the memory is also configured to store a plurality of images, a plurality of classifiers with associated numerical (probability) values and a plurality of thresholds. Further, the memory is preferably configured to store instructions that cause the processor unit according to an aspect of the disclosure to perform the steps of the method of the present disclosure. The memory may be non-transitory and/or comprise a non-transitory memory element. In this context, a non-transitory memory element may include a device that may be tangible, meaning that the device has a concrete physical form, although the device may change its physical state. Thus, for example, non-transitory refers to a device remaining tangible despite a change in state. The memory as mentioned above may be a single memory or may be formed by a plurality of memory elements, wherein said memory elements can be connected to one or more processing units as mentioned above and may be connected to each other and/or said processor units by a local bus system, a local network and/or or a long-distance communication network. Also, the neural networks may be stored on a same or different memory elements than the plurality of images, the plurality of classifiers with associated numerical (probability) values, the plurality of thresholds, and/or the plurality of classification maps, respectively. In other words, the information used in the claimed method may be stored in a centralized or decentralized manner.

The system further comprises a display that is configured to display the CLE images. The display may be integrated in a wall-mounted, ceiling-mounted or standing screen or might be integrated in a video head set that is configured to be worn during surgical operations. The display preferably is configured to display the images with various visually highlighted segments.

The system further comprises the processor unit according to the present disclosure and as described above. Therein the processor unit is further configured to obtain the plurality of images from the CLE unit and/or from the memory, to store (i.e., control the memory to store) the first classifiers, the second classifiers and/or the segmentation maps associated with the corresponding images in the memory. Additionally or alternatively, the processor unit is configured to display (i.e., control the display to display) the second subset of images on the display, wherein at least one image of the second subset of images has at least one graphically highlighted image segment that is determined based on the respective segmentation map. Therein, processor unit is also configured to carry out this determination of the image segments.

In a further preferred embodiment of the system, the CLE unit, the memory and the processor unit are configured to obtain the images of the plurality of images with a given rate, for example a rate of 0.7 to 3 frames per second, preferably a rate up to 10 frames per second, and particularly preferred a rate up to 25 frames per second, and the processor unit is configured to carry out a determination whether one of the plurality of images is one of the second subset of images with an inference time that is less than the time between obtaining subsequent images of the plurality of images. In other words, the processor unit is configured to carry out the steps of the method of the present disclosure (at least until the identifying of the second subset of images, preferably until visualizing the images of the second subset of images with highlighted image segments based on segmentation maps) at the same rate as the images are received from the CLE unit (either directly or indirectly via the memory). Hence, the user does not experience any delay but can be presented - based on the freely selectable user settings - directly with only the filtered and eventually highlighted images. This system advantageously allows to integrate the method into an ongoing surgical operation.

In a further preferred embodiment, the system of the present disclosure further comprises a user interface that is configured to receive a user input, preferably a plurality of user inputs. In this embodiment, the processor unit is further configured to set the at least one first classifiers with the corresponding at least one first thresholds based on a respective user input. The processor unit is further configured to set the at least one second classifiers with the corresponding at least one second thresholds based on a respective user input. Hence, the user is advantageously enabled to freely activate or deactivate the filter stages and to select a filtering strength as described above. The processor unit is further configured to selectively display the first subset of images, the second subset of images or the second subset of images with or without graphically highlighted image segments based on a respective user input. In other words, the user may also select the whole plurality of images for visualisation or may select the first subset of images for visualisation and may also select whether the second subset of images shall be visualized with or without visual highlighted image segments. This advantageously allows the user to better understand the functioning of the system which advantageously improves confidence in the system. These functions for user-selective visualization of images are preferably realized together with a gallery function to store the images for their later review. In other words, the method of the present disclosure can be applied either directly to the received image stream or to images that are already in a storage (gallery). Further, the first and the second neural network can be either applied immediately in sequence or separately based on a user input.

Another aspect of the present disclosure relates to a computer program that is comprising instructions that cause the processor unit of the present disclosure as described above to execute the steps of the method of the present disclosure as described above.

Further aspects of the present invention will be apparent from the dependent claims, the attached drawings and/or the following description of the attached drawings.

### DESCRIPTION OF THE DRAWINGS

Features will become apparent to those of ordinary skill in the art by describing exemplary embodiments in detail with reference to the attached drawings in which:
- Figure 1: schematically illustrates a method according to an embodiment;
- Figure 2: schematically illustrates a method according to an embodiment;
- Figure 3: schematically illustrates a method according to a preferred embodiment;
- Figure 4: schematically illustrates training a first neural network;
- Figure 5: schematically illustrates training a second neural network;
- Figure 6: schematically illustrates training a third neural network;
- Figure 7: schematically illustrates structural features of a first or second neural network;
- Figure 8: schematically illustrates structural features of a third neural network;
- Figure 9: schematically illustrates a method according to a preferred embodiment;
- Figure 10: schematically illustrates a method according to a preferred embodiment;
- Figure 11: schematically illustrates a method according to a preferred embodiment;
- Figure 12: schematically illustrates a displayed image according to an embodiment; and
- Figure 13: schematically illustrates a system according to an embodiment.

### DESCRIPTION OF THE DRAWINGS

Reference will now be made in detail to embodiments which are illustrated in the drawings. Effects and features of the exemplary embodiments will be described with reference to the accompanying drawings. Therein, like reference numerals denote like elements, and redundant descriptions are omitted. The present invention, however, may be embodied in various different forms, and should not be constructed as being limited to only the illustrated embodiments herein. Rather, these embodiments are provided solely as examples for fully conveying the aspects and features of the present invention to those skilled in the art.

Accordingly, processes, elements, and techniques that are not considered necessary to those having ordinary skill in the art for a complete understanding of the aspects and features of the present invention may not be described. At the same time, within the drawings, the relative sizes of elements, layers, and regions may be exaggerated for clarity.

As used herein, the term "and/or" includes any and all combinations of one or more of the associated listed items. Further, the use of "may" when describing embodiments of the present invention refers to "one or more embodiments of the present invention." Further, in the following description of embodiments of the present invention, the terms of a singular form may include plural forms unless the context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. As used herein, the term "and/or" includes any and all combinations of one or more of the associated listed items and expressions such as "at least one of" when preceding a list of elements, modify the entire list of elements.

As used herein, terms such as "substantially" and "about" are used as terms of approximation and not as terms of degree, and are intended to account for the inherent deviations in measured or calculated values that would be recognized by those of ordinary skill in the art. However, if the term "substantially" is used in combination with a feature expressed using a numeric value, the term "substantially" denotes a range of +/- 5% of the value centered on the value.

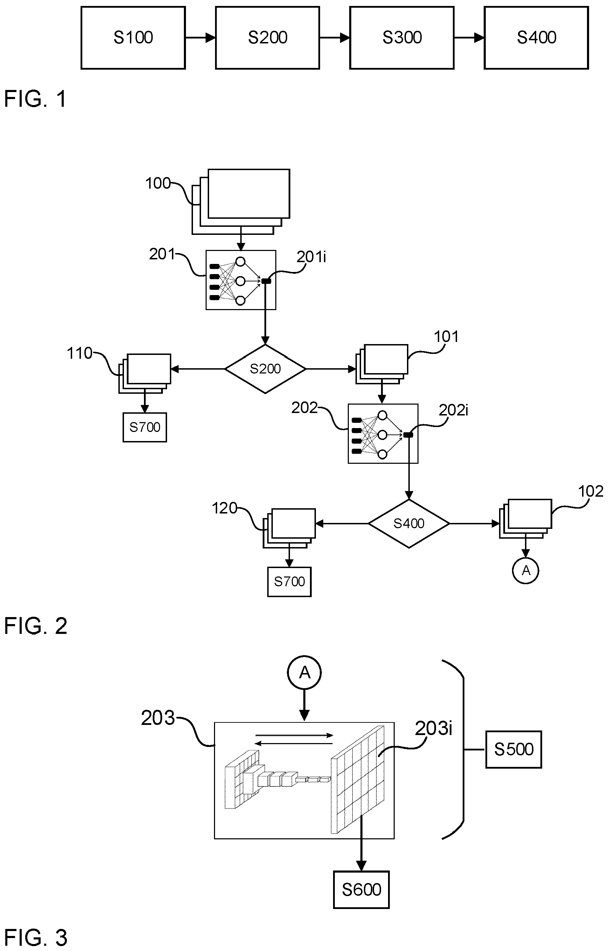

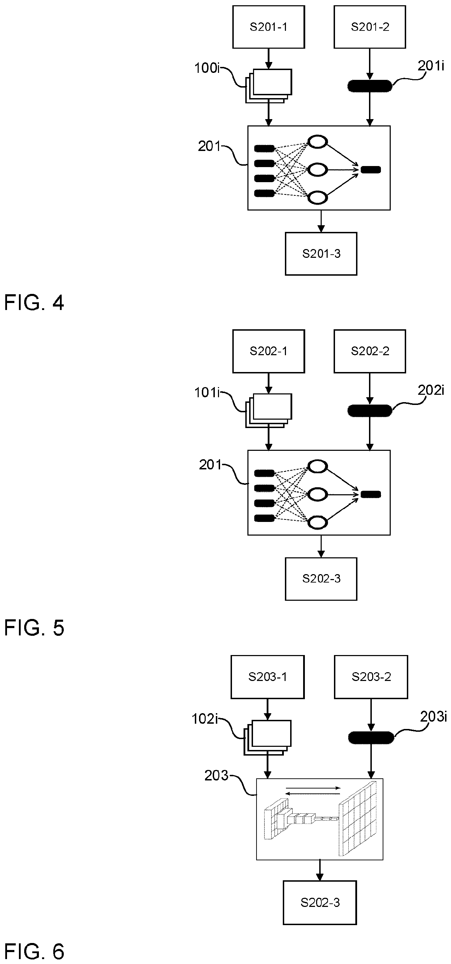

Figures 1 and 2 schematically illustrate a method according to embodiments of the present disclosure. In a first step S100 of the method, each of a plurality of images 100 is input individually into a trained first neural network 201. Therein, the plurality of images 100 were captured using at least one optical device, such as a CLE unit 10. The first trained neural network 201 is trained as explained with respect to Figure 4 and provides, for each image 100i of the plurality of images, a corresponding first classifier 201i indicating the presence of a set tissue distortion type in the image 100i. In a second step S200, the first classifier 201i is compared to a set first threshold in order to determine images that are not affected by image distortion. Particularly, if a first classifier 201i of an image 100i is above a corresponding set first threshold, in step S200 the image is sorted into a subset 110 of low-quality images. These images may be stored in a step S700 for later review but are not further used in the subsequent steps of the method. If a first classifier 201i of an image 100i is below a corresponding set first threshold, in step S200 the image is sorted into the first subset of images 101, which are high quality images. In a third step S300, each image 101i of this first subset of images 101 is input into a trained second neural network 202 that is different from the first neural network 201 and that is trained as explained with respect to Figure 5. The second trained neural network 202 provides for each input image 101i of the first subset of images 101 at least one second classifier 202i as an output that is indicating a presence of a set tissue type in this image 101i. In a fourth step S400, the second classifier 202i is compared to a set second threshold in order to determine images that show a certain tissue type. Particularly, if at least one second classifier 202i of an image 101i is below a corresponding set second threshold, in step S400 the image is sorted into a subset 120 of low relevance images without relevant tissue types. These images may be stored in a step S700 for later review but are not further used in the subsequent steps of the method. If a second classifier 201i of an image 100i is above a corresponding set second threshold, in step S400 the image 101i is sorted into the second subset of images 102, which are medical relevant images, high quality images. These images 102i of the second subset of images 102 can be provided to further method steps (indicated by "A") as illustrated in Figure 3.

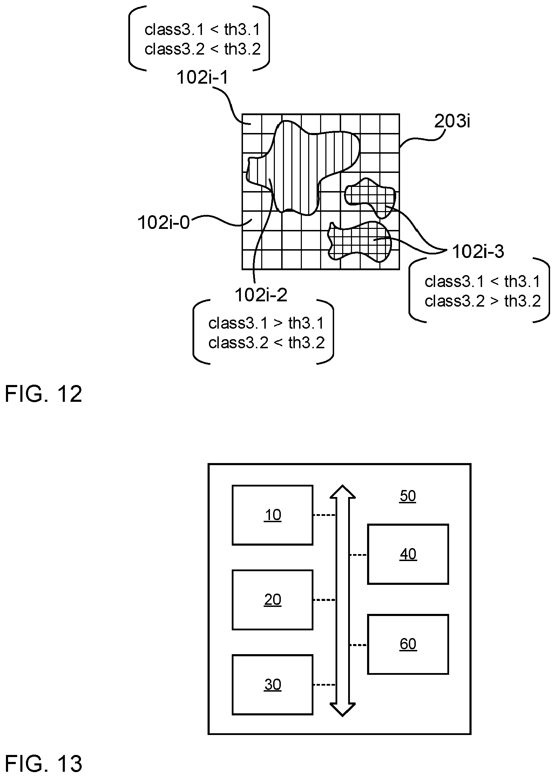

Figure 3 schematically illustrates a method according to a preferred embodiment and starts with receiving the second subset of images 102 determined in the method of Figures 1 and 2. In a fifth step S500, each image 102i of the second subset of images 102 is input individually into a trained third neural network 203 that is different from the first neural network 201 and the second neural networks 202. The third neural network 203 outputs for each image 102i of the second subset of images 102 that was input, a segmentation map 203i that is indicating at least one image segment comprising one of at least one set tissue type. In a further step S600, at least one (preferably all) images 102i of the second subset of images 102 are displayed with at least one visually highlighted image segment (e.g., image segments 102i-1, 102i-2, 102i-3 as illustrated in Figure 12), wherein each of the visually highlighted image's segments is comprising one of the at least one set tissue type and is determined based on the segmentation map 203i.

Figure 4 schematically illustrates the training of a first neural network in order to obtain a first trained neural network 201 as described above. Therein, in step S201-1 a collection (training set) of confocal endomicroscopy images that were captured during neurosurgeries is obtained. In a step S201-2 the collection of images received in step S201-1 are reviewed by neuropathologist(s) who determine the image quality of the reviewed images and classify them considering only the technical quality of the image. Therein, if the reviewed images show indications of a low-quality image like a distorted signal-to-noise-ratio, an optical distortion like movement artefacts, or a reduced contrast or brightness, then they are classified accordingly. Further, if the reviewed images do not show any such indications of a low-quality image then they might be classified as a good quality image by setting a classifier as none-distortion. The images obtained in S201-1 and the classifiers obtained in step S201-2 are then input into a first neural network, here a first convolutional neural network, CNN. The CNN takes these inputs and outputs a set of probabilities of the input image belonging to each of the classification classes. Then, the CNN is trained using an appropriate loss function such as the cross-entropy loss, focal loss, or any other loss function that might be suitable considering the distribution of classified images. Finally, in step 201-3 a first trained neural network 201 is obtained.

Figure 5 schematically illustrates the training of a second neural network in order to obtain a second trained neural network 202 as described above. Therein, in step S202-1 a collection (training set) of high quality confocal endomicroscopy images that were captured during neurosurgeries and that were pre-filtered for image quality is obtained. The pre-filtering can be performed by the first trained neural network 201. In a step S202-2 the collection of images received in step S202-1 are reviewed by neuropathologist(s) who determine the relevance of the reviewed images and classify them considering only the medical relevance of the image. Therein, if the reviewed images contain relevant morphological structures, such as "Necrotic", "Hypercellular", "Giant", "Adenoid", and/or "Fibroid" cellular regions, then they are classified accordingly. Further, if the reviewed images do not show any such indications of medical relevance then they might be classified as a none relevant image by setting a classifier as none-relevant. The images obtained in S202-1 and the classifiers obtained in step S202-2 are then input into a second neural network, here a second convolutional neural network, CNN. The CNN takes these inputs and outputs a set of probabilities of the input image belonging to each of the classification classes. Then, the CNN is trained using an appropriate loss function such as the cross-entropy loss, focal loss, or any other loss function that might be suitable considering the distribution of classified images. Finally, in step S202-3 a second trained neural network 201 is obtained.

Figure 6 schematically illustrates the training of a third neural network in order to obtain a third trained neural network 203 as described above. Therein, in step S203-1 a collection (training set) of high quality and high relevance confocal endomicroscopy images that were captured during neurosurgeries and that were pre-filtered for image quality and medical relevance is obtained. The pre-filtering can be performed by the first trained neural network 201 and the second trained neural network 202. In a step 203-2 the collection of images received in step 203-1 are reviewed by neuropathologist(s) who mark the relevant regions present in the image. Particularly, the neuropathologist(s) identify regions (i.e., pixels or groups of pixels) that are "Hypercellular", "Necrotic", "Giant", "Fibroid", and/or "Adenoid" regions and mark them accordingly, i.e., associate a corresponding classifier with these regions. The images obtained in S203-1 and the markings (i.e., region/pixel information and classifiers) obtained in step 203-2 are then input into a third neural network, here a fully convolutional neural network, FCN. The FCN takes these inputs and outputs, for each received training image, a segmentation marking that is highlighting the "Hypercellular", "Necrotic", "Giant", "Fibroid", and/or "Adenoid" regions present in that image. The FCN is trained using an appropriate loss function such as the cross-entropy loss, focal loss, dice loss, loU loss, Tversky loss or any other loss function that might be suitable for the image segmentation task and the markings received in step S203-2. Finally, in step S203-3 a third trained neural network 203 is obtained.

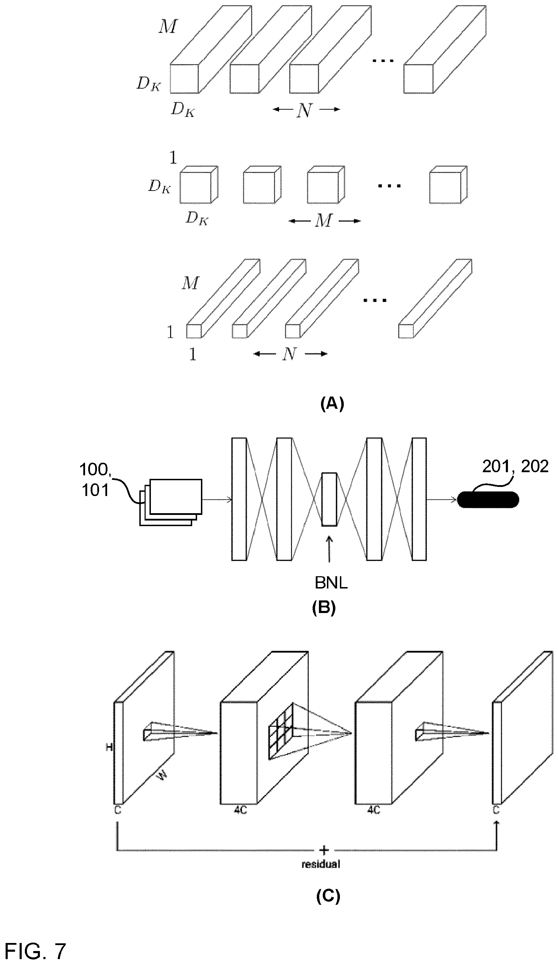

Figure 7 schematically illustrates structural features of a first or a second neural network.

Figure 7(A) schematically illustrates a depthwise separable convolution. The uppermost image of Figure 7(A) shows a standard convolutional filter that takes as input a *D_{F}* * *D_{F} * M* feature map and produces a *D_{G}* * *D_{G}* * *N* feature map, where *D_{F}* is the spatial width and height of a square input feature map, M is the number of input channels (input depth), *D_{G}* is the spatial width and height of a square output feature map and N is the number of output channels (output depth). Further, the standard convolutional layer is parameterized by convolution kernel of size *D_{K} * D_{K} * M* * *N* where *D_{K}* is the spatial dimension of the kernel assumed to be square, *M* is number of input channels and *N* is the number of output channels. Assuming a stride one and padding, the computational cost for such standard convolution is *D_{K} *D_{K} *M *N *D_{F} *D_{F},* i.e., the computational cost depends multiplicatively on the number of input channels *M,* the number of output channels *N* the kernel size *D_{K} * D_{K}* and the feature map size *D_{F} * D_{F}.*

Depthwise separable convolution is made up of two layers: the depthwise convolution as shown in the middle image of Figure 7(A) and pointwise convolutions as show in the lowermost image of Figure 7(A). The depthwise convolutions apply a single filter per each input channel (input depth), while the 1 * 1 pointwise convolution is used to create a linear combination of the output of the depthwise layer. Depthwise convolution with one filter per input channel (input depth) where a depthwise convolutional kernel of size *D_{K} * D_{K} *M* is applied to each channel has a computational cost of *D_{K} * D_{K} * M * D_{F} * D_{F}.* Depthwise convolution filters input channels but does not combine them to create new features and thus the additional 1* 1 pointwise convolution is added that computes a linear combination of the output of depthwise convolution via 1 × 1 convolution to generate these new features. The combination of these two convolutions is called depthwise separable convolution and has the computational cost of *D_{K}* * *D_{K}* * *M* * *D_{F}* * *D_{F}* + *M* * *N* * *D_{F}* * *D_{F}* which is less than the computational cost of the standard convolution.

Figure 7(B) schematically illustrates a linear bottleneck layer, BNL, in a neural network receiving images 100, 101 and outputting classifiers, such as first and second classifiers 201, 202. In a deep neural network consisting of n layers a set of layer activations may form a manifold of interest that could be embedded in low-dimensional subspaces. This can be exploited by simply reducing the dimensionality of a layer thus reducing the dimensionality of the operating space. Figure 7(B) shows the reduced dimensionality of such a bottleneck layer, BNL. However, when non-linear activation functions, such as ReLU, are used in neural networks that e.g., discard values that are smaller than 0, there is a loss of information that can be only tackled by increasing the number of channels in order to increase the capacity of the network. Hence, in the first trained neural network 201 and the second trained neural network 202 preferably linear bottleneck layers are used where the last convolution of a residual block as explained below has a linear output before being added to the initial activations. A linear bottleneck layer can be realized by a 1* 1 pointwise convolution as described above for reducing the size of an input.

Figure 7(C) schematically illustrates an inverted residual layer. The idea of residual connections is to add an input to an output such that an output becomes a layer function of an input plus an input. Such residual operation improves the ability of a gradient to propagate across multiplier layers and hence permits effectively training of networks with a high number of layers. As illustrated in Figure 7(C) inverted residual layers can be considered to be inverted bottleneck layers as they expand features with a first convolution instead of reducing them. The inverted residual layers thus establish a residual connection between the bottleneck layers of low dimension (instead between layers of high dimension) storing the features of interest.

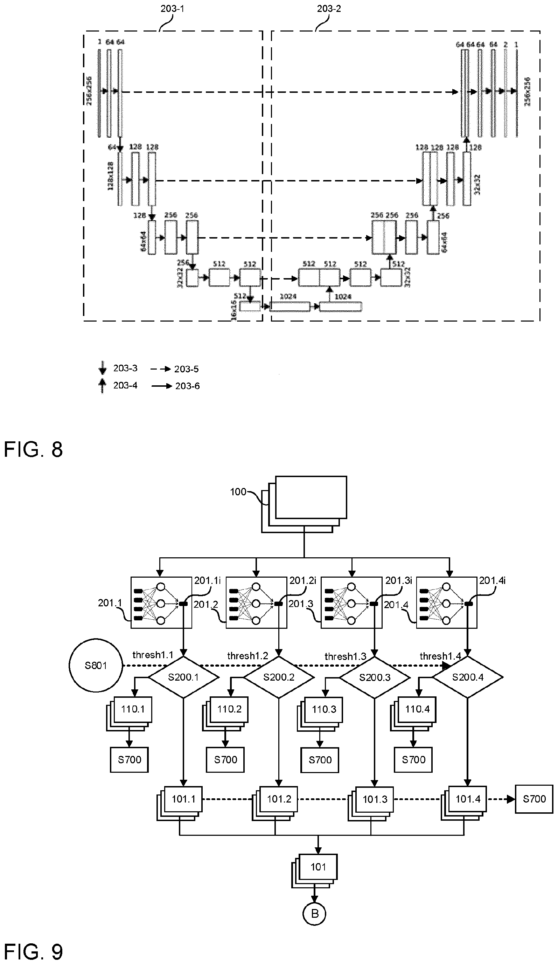

Figure 8 schematically illustrates structural features of a third neural network 203. As illustrated, a third neural network 203 comprises a contracting subnetwork 203-1 and an expanding subnetwork 203-2. Therein, the contracting paths in the contracting subnetwork 203-1 are classical convolution paths where a resolution of an input image shrinks the deeper into the layers it gets while the number of channels increases. Further, the expanding paths in the expanding subnetwork 203-2 are the inverse of the down paths and increase the resolution of an image again by upscaling the image size while reducing the number of channels. As further illustrated in Figure 8, the contracting subnetwork 203-1 and the expanding subnetwork 203-2 are connected on each level by a direct connection, so-called concatenations 203-5.

As illustrated in Figure 8 an input image may be of a resolution 256 * 256 and in one level of the contracting subnetwork 203-1 a two-dimensional convolution 203-6 with a kernel size 3 and padding is applied two times, each followed by a rectified linear unit, ReLU (not shown), and a 2x2 max pooling operation 203-3 with stride 2 is applied one time for downsampling. At each downsampling step, the number of feature channels is doubled. One level in the expanding subnetwork 203-2 consists of an 2x2 upsampling operation 203-4 of the feature map followed by a convolution 203-6 ("up-convolution") that halves the number of feature channels and a concatenation with the correspondingly feature map from the contracting path, and two 3x3 convolutions, each followed by a ReLU. In order to further improve the efficiency of the third neural network data, augmentation may be applied to the training data in the training as described with respect to Figure 7, particularly augmentation (i.e., creating more training images) by shifting the image a few pixels to either side, by rotating the image by a few degrees, by elastic deformations of the image, e.g., by applying a 3x3 pixel grid of random displacements with bicubic per-pixel displacements or by changing the color or grey scales of the images.

Figure 9 schematically illustrates a method according to a preferred embodiment. Therein, the first neural network 201 comprises a plurality of first neural subnetworks 201.1, 201.2, 201.3, 201.4, each of which receives the plurality of images 100 and outputs, for each image 100i of the plurality of images 100, one of a plurality of first classifiers 201.1i, 201.2i, 201.3i, 201.4i. Therein, each of these first classifiers 201.1i, 201.2i, 201.3i, 201.4i is output with a respective value indicating a probability of a certain image distortion type 1 to 4 being present in the image. In each path of the method as illustrated in Figure 9, the first classifiers 201.1i, 201.2i, 201.3i, 201.4i are then individually compared to corresponding first thresholds thresh1.1, thresh1.2, thresh1.3, thresh1.4 that are individually set based on corresponding user inputs in step S801. In each path, images with a first classifier 201.1i, 201.2i, 201.3i, 201.4i above the corresponding first threshold thresh1.1, thresh1.2, thresh1.3, thresh1.4 are sorted as low-quality images 110.1, 110.2, 110.3, 110.4 and may be stored in step S700 for later review. Images with a first classifier 201.1i, 201.2i, 201.3i, 201.4i below the corresponding first threshold thresh1.1, thresh1.2, thresh1.3, thresh1.4 are sorted as respective high-quality images 101.1, 101.2, 101.3, 101.4 and may be individually stored in step S700 for later review. A first subset of images 101 is then determined based on high quality images 101.1, 101.2, 101.3, 101.4, e.g., by selecting only images that are present in each of the high-quality images 101.1, 101.2, 101.3, 101.4. In this embodiment, the user can advantageously activate, deactivate, and adjust individual paths.

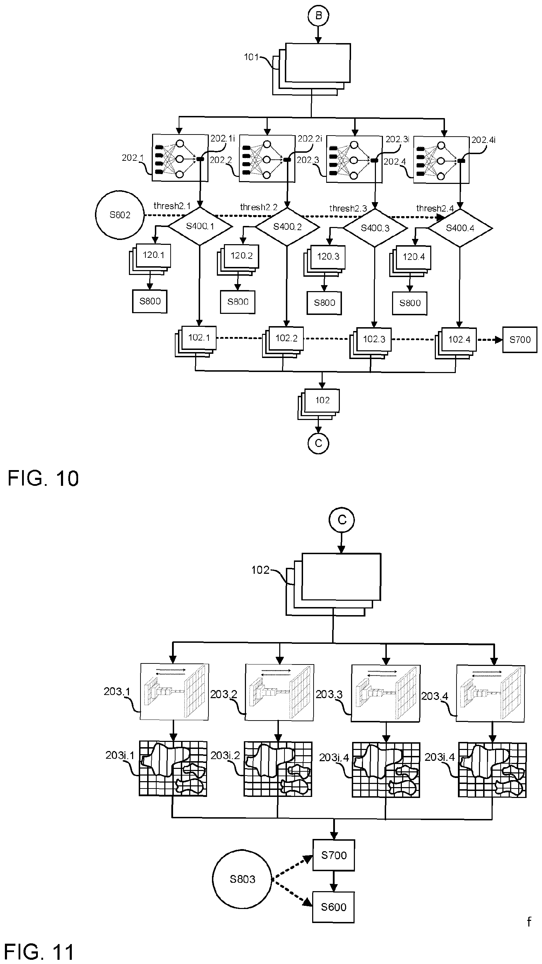

Figure 10 schematically illustrates a method according to a preferred embodiment. Therein, a first subset of images 101 is received, e.g., as determined in the method of Figure 9. In the method of Figure 10 the second neural network 202 comprises a plurality of second neural subnetworks 202.1, 202.2, 202.3, 202.4, each of which receives the first subset of images 101 and outputs, for each image 101i of the first subset of images 101, one of a plurality of second classifiers 202.1i, 202.2i, 202.3i, 202.4i. Therein, each of these second classifiers 202.1i, 202.2i, 202.3i, 202.4i is output with a respective value indicating a probability of a certain tissue type 1 to 4 being present in the image. In each path of the method as illustrated in Figure 10, the second classifiers 202.1i, 202.2i, 202.3i, 202.4i are then individually compared to corresponding second thresholds thresh2.1, thresh2.2, thresh2.3, thresh2.4 that are individually set based on corresponding user inputs in step S802. In each path, images with a second classifier 202.1i, 202.2i, 202.3i, 202.4i below the corresponding second threshold thresh2.1, thresh2.2, thresh2.3, thresh2.4 are sorted as low relevance images 120.1, 120.2, 120.3, 120.4 and may be stored in step S700 for later review. Images with a second classifier 202.1i, 202.2i, 202.3i, 202.4i above the corresponding second threshold thresh2.1, thresh2.2, thresh2.3, thresh2.4 are sorted as respective high relevance images 102.1, 102.2, 102.3, 102.4 and may be individually stored in step S700 for later review. A second subset of images 102 is then determined based on the high relevance images 102.1, 102.2, 102.3, 102.4, e.g., by selecting only images that are present in exactly one of the high relevance images 102.1, 102.2, 102.3, 102.4. In this embodiment, the user can advantageously activate, deactivate, and adjust individual paths.

Figure 11 schematically illustrates a method according to a preferred embodiment. Therein, a second subset of images 102 is received, e.g., as determined in the method of Figure 10. In the method of Figure 11 the third neural network 203 comprises a plurality of third neural subnetworks 203.1, 203.2, 203.3, 203.4, each of which receives the second subset of images 102 and outputs, for each image 102i of the second subset of images 102, a segmentation map 203.1i, 203.2i, 203.3i, 203.4i indicating at least one image segment 102i-1, 102i-2, 102i-3 comprising one of a plurality of set tissue types. Therein, each segmentation map 203.1i, 203.2i, 203.3i, 203.4i comprises, for each pixel 102i-0 or group of pixels of the image 102i, one of a plurality of third classifiers related to a presence of the one of the plurality of set tissue types in the respective pixel 102i-0 or group of pixels. The combination of images 102i of the second subset of images 102 with the respective segmentation maps 203.1i, 203.2i, 203.3i, 203.4i can then be stored in step S700 for later review based on corresponding user inputs in step S803. Further, an image 102i of the second subset of images 102 can be displayed in step S600 with at least one visually highlighted image segment 102i-1, 102i-2, 102i-3 comprising one of the at least one set tissue type based on at least one of the respective segmentation maps 203.1i, 203.2i, 203.3i, 203.4i.

Figure 12 schematically illustrates such a displayed image 102i according to an embodiment. Therein, the image 102i is displayed based on a segmentation map 203i. This segmentation map 203i may be one or a combination of the segmentation maps 203.1i, 203.2i, 203.3i, 203.4i, particularly of two segmentation maps 203.1i, 203.2i determined in the method of Figure 11. As illustrated in Figure 12, the image 102i comprise three different image segments 102i-1, 102i-2, 102i-3, which are distinguished by having different values of the respective third classifiers class3.1 and class3.2 vis-à-vis the third thresholds th3.1 and th3.2. Particularly, the first image segment 102i-1 consists of pixels 102i-0 for which both third classifiers class3.1 and class3.2 are smaller than the respective third thresholds th3.1 and th3.2. Hence, the pixels 102i-0 of the first image segment 102i-1 have been found by the third trained neural network 203 to comprise neither a tissue type associated with the third classifier class 3.1 (e.g., "giant cells") or with the third classifier class3.2 (e.g., "fibroid cells"). On the other hand, the second image segment 102i-2 consists of pixels 102i-0 for which the third classifiers class3.1 is larger than the respective third threshold th3.1, while the third classifier class3.2 is smaller than the respective third threshold th3.2. Hence, the second image segment 102i-2 was identified by the third trained neural network 203 to comprise "giant cells" but no "fibroid cells" and the second image segment 102i-2 is highlighted accordingly, e.g., by overlaying the region with a first color. The third image segment 102i-3 consists of pixels 102i-0 for which the third classifiers class3.1 is smaller than the respective third threshold th3.1, while the third classifier class3.2 is larger than the respective third threshold th3.2. Hence, the third image segment 102i-2 was identified by the third trained neural network 203 to comprise "fibroid cells" but no "giant cells" and the third image segment 102i-3 is highlighted accordingly, e.g., by overlaying the region with a second color.

Figure 13 schematically illustrates a system 50 for processing images. The system 50 comprises a confocal laser endomicroscopy, CLE, unit 10 that is configured for capturing a plurality of CLE images 100, a memory 20 that is configured for storing at least one trained neural network 201, 202, 203 and a display 30 that is configured to display the CLE images 100. The system 50 further comprises a processor unit 40 that is configured to obtain a plurality of images 100 captured using at least one optical device from the CLE unit 10 and/or from the memory 20, to input the plurality of images 100 into a trained first neural network 201 and to receive, as an output of the first neural network 201 and for each of the plurality of images 100, at least one first classifier 201i indicating a set image distortion type of the image 100i, to identify a first subset 101 of the plurality of images 100 based on the first classifiers 201i, to input the first subset of images 101 into a trained second neural network 202 different from the first neural network 201 and to receive, as an output of the second neural network 202 and for each of the first subset of images 101, at least one second classifier 202i indicating a presence of a set tissue type in the image 101i; and to identify a second subset 102 of the first subset of images 101 based on the second classifiers 202i. Further, the processor unit may further be configured to store the first classifiers 201i, the second classifiers 202i and/or the segmentation maps 203i associated with the corresponding images in the memory 20, and/or to display the second subset of images 102 on the display 30, wherein at least one image 102i of the second subset of images 102 has at least one graphically highlighted image segment 102i 1, 102i 2, 102i 3 that is determined based on the respective segmentation map 203i.

### REFERENCE SIGNS

- 10: confocal laser endomicroscopy, CLE, unit
- 20: memory
- 30: display
- 40: processor unit
- 50: system
- 60: user interface

- 100: plurality of images
- 100i: specific image
- 101: first subset of images
- 101i: specific image of first subset
- 102: second subset of images
- 102i: specific image of second subset
- 102i-0: pixel
- 102i-1...3: image segments
- 110: low quality images
- 120: low significance images

- 201: first neural network
- 201.1... 4: first neural subnetworks
- 202: second neural network
- 202.1...4: second neural subnetworks
- 203: third neural network
- 203.1... 4: third neural subnetworks
- 203-1: contracting subnetwork
- 203-2: expansive subnetwork
- 203-3: pooling operator
- 203-4: upsampling operator
- 203-5: concatenations
- 203-6: convolution
- 201i: first classifiers
- 202i: second classifiers
- 203i: segmentation maps
- S100: inputting plurality of images in first neural network
- S201: training a first neural network
- S202: training a second neural network
- S203: training a third neural network
- S200: identifying a first subset of images
- S300: inputting first subset of images in second neural network
- S400: identifying a second subset of images
- S500: inputting second subset of images in third neural network
- S600: displaying image of the second subset with a visually highlighted image segment
- S700: storing data (images/classifiers/segmentation map)
- S801...3: receiving user input

## Claims

1. Method for processing of images (100, 101, 102), the method comprising the steps of:
inputting (S100) a plurality of images (100) captured using at least one optical device into a trained first neural network (201) and receiving, as an output of the first neural network (201) and for each of the plurality of images (100), at least one first classifier (201i) indicating a set image distortion type of the image (100i);
identifying (S200) a first subset (101) of the plurality of images (100) based on the first classifiers (201i);
inputting (S300) the first subset of images (101) into a trained second neural network (202) different from the trained first neural network (201) and receiving, as an output of the second neural network (202) and for each of the first subset of images (101), at least one second classifier (202i) indicating a presence of a set tissue type in the image (101i); and
identifying (S400) a second subset (102) of the first subset of images (101) based on the second classifiers (202i).

2. Method for processing of images (100, 101, 102), the method comprising the steps of:
identifying a first subset (101) of a plurality of images (100) that is based on first classifiers (201i) output by a trained first neural network (201) for the plurality of images (100), the first classifiers (201i) being related to image distortion types of the images (100);
inputting the first subset of images (101) into a trained second neural network (202) different from the trained first neural network (201) and receiving, as an output of the second neural network (202) and for each of the first subset of images (101), at least one second classifier (202i) indicating a presence of a set tissue type in the image (101i); and
identifying (S5400) a second subset (102) of the first subset of images (101) based on the second classifiers (202i).

3. Method for processing of images (100, 101, 102), the method comprising the steps of:
inputting a first subset (101) of a plurality of images (100) into a trained second neural network (202) different from a trained first neural network (201), wherein the first subset of images (101) is identified based on first classifiers (201i) that are output by the trained first neural network (201) with respect to each of the plurality of images (100),
receiving, as an output of the second neural network (202) and for each of the first subset of images (101), at least one second classifier (202i); and
identifying (S5400) a second subset (102) of the first subset of images (101) based on the second classifiers (202i),
wherein the first classifiers (201i) are related to image distortion types of the plurality of images (100) and wherein the second classifiers (202i) are related to a presence of a set tissue type in the first subset of images (101).

4. Method according to any one of claims 1 to 3, further comprising the step of obtaining a plurality of images (100) captured using at least one optical device, preferably captured using one of a confocal laser endomicroscopy, CLE, unit, a confocal microscope, or a surgical microscope or endoscope with a high-resolution camera.

5. Method according to any one of the preceding claims, wherein
each image (101i) of the first subset of images (101) is associated with a respective first classifier (201i) indicating one of the at least one set image distortion type with a probability below a set first threshold, and/or
each image (102i) of the second subset of images (102) is associated with a respective second classifier (202i) indicating a presence of one of the at least one set tissue type with a probability above a set second threshold.

6. Method according to any one of the preceding claims, wherein
each first classifier (201i) indicates an image distortion type being one of an optical distortion, a diminished signal-to-noise-ratio, dSNR, an irregular brightness or contrast, and a non-distortion, and/or
each second classifier (202i) indicates a present tissue type being one of a hypercellular region, a necrotic region, a fibroid region, an adenoid region, and a giant cell region.

7. Method according to claim 5 or 6, wherein the at least one first classifier (201i) with the corresponding at least one first threshold and/or the at least one second classifier (202i) with the corresponding at least one second threshold are set based on a user input.

8. Method according to any one of the preceding claims, wherein at least one of the first (201) and the second neural network (202) is a convolutional neural network, CNN, using depthwise separable convolution, linear bottleneck layers and/or inverted residual layers.

9. Method according to any one of the preceding claims, further comprising the steps of:
inputting (S500) the second subset of images (102) into a trained third neural network (203) different from the first neural network (201) and the second neural network (202);
receiving, as an output of the third neural network (203) and for each of the second subset of images (102), a segmentation map (203i) indicating at least one image segment (102i-1, 102i-2, 102i-3) comprising one of the at least one set tissue type in the image (102i).

10. Method according to claim 9, wherein the segmentation map (203i) comprises, for each pixel (102i-0) or group of pixels (102i-0) of the respective image (102i), at least one third classifier related to a presence of one of the at least one set tissue type in the respective pixel (102i-0) or group of pixels (102i-0).

11. Method according to claim 9 or 10, further comprising the step of: displaying (S600) an image (102-i) of the second subset of images (102) with at least one visually highlighted image segment (102i-1, 102i-2, 102i-3) comprising one of the at least one set tissue type based on the segmentation map (203i).

12. Method according to any one of claims 9 to 11, wherein the third neural network (203) is a fully convolutional neural network, FCN, comprising a contracting subnetwork (203-1) with a plurality of pooling operators (203-3) followed by an expansive subnetwork (203-2) with a plurality of upsampling operators (203-4) and a plurality of concatenations (203-5) connecting each layer of the contracting subnetwork (203-1) with a corresponding layer of the expansive subnetwork (203-2).

13. Method according to any one of the preceding claims, wherein
the first neural network (201) comprises a plurality of first neural subnetworks (201.1...201.4), each of which receives the plurality of images (100) and outputs, for each of the plurality of images (100), one of a plurality of first classifiers (201.1i...201.4i) with a respective probability, and/or
the second neural network (202) comprises a plurality of second neural subnetworks (202.1 ...202.4), each of which receives the first subset of images (101) and outputs, for each of the subset of images (11), one of a plurality of second classifiers (202.2i...202.4i) with a respective probability, and/or
method according to any one of claims 9 to 12, wherein
the third neural network (203) comprises a plurality of third neural subnetworks (203.1 ... 203.4), each of which receives the second subset of images (102) and outputs, for each of the subset of images (102), a segmentation map (203.1i...203.4i) indicating at least one image segment (102i-1, 102i-2, 102i-3) comprising one of a plurality of set tissue types, wherein the segmentation map (203.1i...203.4i) comprises, for each pixel (102i-0) or group of pixels of the image (102i), one of a plurality of third classifiers related to a presence of the one of the plurality of set tissue types in the respective pixel (102i-0) or group of pixels.

14. Processor unit (40) configured for performing the method of any one of the claims 1 to 13.

15. System (50) for processing images, comprising:
a confocal laser endomicroscopy, CLE, unit (10) configured for capturing a plurality of CLE images (100);
a memory (20) configured for storing at least one trained neural network (201, 202, 203);
a display (30) configured to display the CLE images (100); and
the processor unit (40) of claim 14, wherein the processor unit (40) is further configured to:
obtain the plurality of images from the CLE unit (10) and/or from the memory (20),
store the first classifiers (201i), the second classifiers (202i) and/or the segmentation maps (203i) associated with the corresponding images in the memory (20), and/or
display the second subset of images (102) on the display (30), wherein at least one image (102i) of the second subset of images (102) has at least one graphically highlighted image segment (102i-1, 102i-2, 102i-3) determined based on the respective segmentation map (203i).

16. System of claim 15, wherein the CLE unit (10), the memory (20) and the processor unit (40) are configured to obtain the images (100i) of the plurality of images (100) with a rate defining a time between obtaining subsequent images of the plurality of images (100) and wherein the processor unit (40) is configured to carry out a determination whether one of the plurality of images (100) is one of the second subset of images (102) with an inference time that is less than the time between obtaining subsequent images of the plurality of images (100).

17. System of claim 15 or 16, further comprising a user interface (60) configured to receive a user input, wherein the processor unit (40) is configured to, each based on a respective user input:
set the at least one first classifiers (201i) with the corresponding at least one first thresholds,
set the at least one second classifiers (202i) with the corresponding at least one second thresholds, and/or
selectively display the first subset of images (101), the second subset of images (102) or the second subset of images (102) with or without graphically highlighted image segments (102i-1, 102i-2, 102i-3).

18. A computer program comprising instructions to cause the processor unit (40) of claim 14 to execute the steps of the method of any one of claims 1 to 13.

## Amended claims

### Amended claims in accordance with Rule 137(2) EPC.

1. Method for processing of images (100, 101, 102), the method comprising the steps of:
inputting a first subset (101) of a plurality of images (100) into a trained second neural network (202) different from a trained first neural network (201), wherein the first subset of images (101) is identified based on first classifiers (201i) that are output by the trained first neural network (201) with respect to each of the plurality of images (100),
receiving, as an output of the second neural network (202) and for each of the first subset of images (101), at least one second classifier (202i);
identifying (S5400) a second subset (102) of the first subset of images (101) based on the second classifiers (202i);
inputting (S500) the second subset of images (102) into a trained third neural network (203) different from the first neural network (201) and the second neural network (202); and
receiving, as an output of the third neural network (203) and for each of the second subset of images (102), a segmentation map (203i) indicating at least one image segment (102i-1, 102i-2, 102i-3) comprising one of the at least one set tissue type in the image (102i),
wherein the first classifiers (201i) are related to image distortion types of the plurality of images (100) and wherein the second classifiers (202i) are related to a presence of a set tissue type in the first subset of images (101), and
wherein the segmentation map (203i) comprises, for each pixel (102i-0) or group of pixels (102i-0) of the respective image (102i), at least one third classifier related to a presence of one of the at least one set tissue type in the respective pixel (102i-0) or group of pixels (102i-0).

2. Method according to claim 1, further comprising the step of identifying the first subset (101) of a plurality of images (100) based on the first classifiers (201i) output by the trained first neural network (201) for the plurality of images (100).

3. Method according to claim 1 or 2, wherein the plurality of images (100) are captured using at least one optical device and the method further comprises the step of inputting (S100) the plurality of images (100) into the trained first neural network (201) and receiving, as an output of the first neural network (201) and for each of the plurality of images (100), at least one first classifier (201i) indicating a set image distortion type of the image (100i).

4. Method according to any one of claims 1 to 3, further comprising the step of obtaining a plurality of images (100) captured using at least one optical device, preferably captured using one of a confocal laser endomicroscopy, CLE, unit, a confocal microscope, or a surgical microscope or endoscope with a high-resolution camera.

5. Method according to any one of the preceding claims, wherein
each image (101i) of the first subset of images (101) is associated with a respective first classifier (201i) indicating one of the at least one set image distortion type with a probability below a set first threshold, and/or
each image (102i) of the second subset of images (102) is associated with a respective second classifier (202i) indicating a presence of one of the at least one set tissue type with a probability above a set second threshold.

6. Method according to any one of the preceding claims, wherein
each first classifier (201i) indicates an image distortion type being one of an optical distortion, a diminished signal-to-noise-ratio, dSNR, an irregular brightness or contrast, and a non-distortion, and/or
each second classifier (202i) indicates a present tissue type being one of a hypercellular region, a necrotic region, a fibroid region, an adenoid region, and a giant cell region.

7. Method according to claim 5 or 6, wherein the at least one first classifier (201i) with the corresponding at least one first threshold and/or the at least one second classifier (202i) with the corresponding at least one second threshold are set based on a user input.

8. Method according to any one of the preceding claims, wherein at least one of the first (201) and the second neural network (202) is a convolutional neural network, CNN, using depthwise separable convolution, linear bottleneck layers and/or inverted residual layers.

9. Method according to claim 7 or 8, further comprising the step of: displaying (S600) an image (102-i) of the second subset of images (102) with at least one visually highlighted image segment (102i-1, 102i-2, 102i-3) comprising one of the at least one set tissue type based on the segmentation map (203i).

10. Method according to any one of claims 7 to 9, wherein the third neural network (203) is a fully convolutional neural network, FCN, comprising a contracting subnetwork (203-1) with a plurality of pooling operators (203-3) followed by an expansive subnetwork (203-2) with a plurality of upsampling operators (203-4) and a plurality of concatenations (203-5) connecting each layer of the contracting subnetwork (203-1) with a corresponding layer of the expansive subnetwork (203-2).

11. Method according to any one of the preceding claims, wherein
the first neural network (201) comprises a plurality of first neural subnetworks (201.1...201.4), each of which receives the plurality of images (100) and outputs, for each of the plurality of images (100), one of a plurality of first classifiers (201.1i...201.4i) with a respective probability, and/or
the second neural network (202) comprises a plurality of second neural subnetworks (202.1...202.4), each of which receives the first subset of images (101) and outputs, for each of the subset of images (11), one of a plurality of second classifiers (202.2i... 202.4i) with a respective probability, and/or
method according to any one of claims 7 to 10, wherein
the third neural network (203) comprises a plurality of third neural subnetworks (203.1...203.4), each of which receives the second subset of images (102) and outputs, for each of the subset of images (102), a segmentation map (203.1i...203.4i) indicating at least one image segment (102i-1, 102i-2, 102i-3) comprising one of a plurality of set tissue types, wherein the segmentation map (203.1i... 203.4i) comprises, for each pixel (102i-0) or group of pixels of the image (102i), one of a plurality of third classifiers related to a presence of the one of the plurality of set tissue types in the respective pixel (102i-0) or group of pixels.

12. Processor unit (40) configured for performing the method of any one of the claims 1 to 11.

13. System (50) for processing images, comprising:
a confocal laser endomicroscopy, CLE, unit (10) configured for capturing a plurality of CLE images (100);
a memory (20) configured for storing at least one trained neural network (201, 202, 203);
a display (30) configured to display the CLE images (100); and
the processor unit (40) of claim 12, wherein the processor unit (40) is further configured to:
obtain the plurality of images from the CLE unit (10) and/or from the memory (20),
store the first classifiers (201i), the second classifiers (202i) and/or the segmentation maps (203i) associated with the corresponding images in the memory (20), and/or
display the second subset of images (102) on the display (30), wherein at least one image (102i) of the second subset of images (102) has at least one graphically highlighted image segment (102i-1, 102i-2, 102i-3) determined based on the respective segmentation map (203i).

14. System of claim 13, wherein the CLE unit (10), the memory (20) and the processor unit (40) are configured to obtain the images (100i) of the plurality of images (100) with a rate defining a time between obtaining subsequent images of the plurality of images (100) and wherein the processor unit (40) is configured to carry out a determination whether one of the plurality of images (100) is one of the second subset of images (102) with an inference time that is less than the time between obtaining subsequent images of the plurality of images (100).

15. System of claim 13 or 14, further comprising a user interface (60) configured to receive a user input, wherein the processor unit (40) is configured to, each based on a respective user input:
set the at least one first classifiers (201i) with the corresponding at least one first thresholds,
set the at least one second classifiers (202i) with the corresponding at least one second thresholds, and/or
selectively display the first subset of images (101), the second subset of images (102) or the second subset of images (102) with or without graphically highlighted image segments (102i-1, 102i-2, 102i-3).

16. A computer program comprising instructions to cause the processor unit (40) of claim 12 to execute the steps of the method of any one of claims 1 to 11.
